# EUROPÄISCHE PATENTSCHRIFT

(11) **EP 3 986 174 B1**
(45) Veröffentlichungstag und Bekanntmachung des Hinweises auf die Patenterteilung: **06.11.2024**
(21) Anmeldenummer: 20733747.8
(22) Anmeldetag: 17.06.2020
(51) Int. Cl.: A24F 40/10, A24F 40/57, A24F 40/42, A24F 40/44, A61M 11/04, A61M 15/06, A61M 16/00

(54) **VERDAMPFERKARTUSCHE SOWIE INHALATOR MIT EINER SOLCHEN VERDAMPFERKARTUSCHE**
VAPORIZER CARTRIDGE AND INHALER COMPRISING SUCH A VAPORIZER CARTRIDGE
CARTOUCHE DE VAPORISATION ET INHALATEUR POURVU D'UNE TELLE CARTOUCHE DE VAPORISATION

(30) Priorität: 20.06.2019 DE 102019116728
(43) Veröffentlichungstag der Anmeldung: 27.04.2022
(73) Patentinhaber: Körber Technologies GmbH, 21033 Hamburg (DE)
(72) Erfinder: BERGMANN, Max, 22303 Hamburg (DE); CORNILS, Lasse, 22605 Hamburg (DE); GIESE, Matthias, Tokyo 104-0053 (JP); HANNEKEN, Christian, 22299 Hamburg (DE); JAKLIN, Jan, 73054 Eislingen (DE); KESSLER, Marc, 22415 Hamburg (DE); KLEINE WÄCHTER, Michael, 23881 Lankau (DE); MÜLLER, Thomas, 20259 Hamburg (DE); ROMMING, Niklas, 24306 Plön (DE); SCHMIDT, Rene, 21244 Buchholz i.d.N. (DE); SCHUSTER, Christof, 20259 Hamburg (DE); WUTTKE, Tobias, 21465 Reinbek (DE)
(74) Vertreter: Stork Bamberger Patentanwälte PartmbB
(86) Internationale Anmeldenummer: PCT/EP2020/066737
(87) Internationale Veröffentlichungsnummer: WO 2020/254390

(56) Entgegenhaltungen:
- EP-A1- 3 417 726
- EP-A2- 3 200 559
- WO-A1-2013/083638
- WO-A1-2018/211035
- DE-A1- 102017 123 870
- GB-A- 2 561 867
- US-A1- 2016 316 819
- US-A1- 2017 367 411

## Beschreibung

Die Erfindung betrifft eine Verdampferkartusche als Bestandteil eines Inhalators, umfassend einen Hohlkörper mit einem durchgängigen Strömungskanal sowie einen Vorratstank zum Bevorraten von Flüssigkeit, wobei der Vorratstank mindestens eine Zugangsöffnung zum Strömungskanal aufweist und im Bereich jeder Zugangsöffnung eine sich über die gesamte Zugangsöffnung erstreckende Verdampfereinheit angeordnet ist, die ein Dochtorgan und ein Heizorgan aufweist, wobei die Verdampfereinheit flüssigkeitspermeabel ausgebildet ist, derart, dass Flüssigkeit mindestens initial kapillar aus dem Vorratstank durch die Verdampfereinheit in Richtung des Strömungskanals förderbar ist.

Des Weiteren betrifft die Erfindung einen Inhalator, ausgebildet und eingerichtet zum Inhalieren von mit Wirkstoffen angereichertem Dampf, umfassend einen mindestens eine elektronische Steuereinheit und eine Energiequelle umfassenden Kartuschenträger sowie eine Verdampferkartusche.

Solche Verdampferkartuschen und Inhalatoren kommen in der Genussmittelindustrie, hier insbesondere im Zusammenhang mit einer elektronischen Zigarette, der so genannten E-Zigarette, sowie im medizinischen Bereich zum Einsatz, um fluide Genussmittel und/oder fluide medizinische Produkte in Dampfform und/oder als Aerosole inhalieren zu können. Beim Konsumieren saugt üblicherweise eine Person an einem Mundstück des Inhalators, wodurch in dem Strömungskanal ein Saugdruck entsteht, der einen Luftstrom durch den Strömungskanal erzeugt. Der Luftstrom kann aber auch maschinell z.B. durch eine Pumpe erzeugt werden. In dem Strömungskanal wird dem Luftstrom eine von der Verdampfereinheit erzeugte und bereitgestellte verdampfte Flüssigkeit zugegeben, um der konsumierenden Person ein Aerosol oder ein Aerosol-Dampf-Gemisch zu verabreichen. Die Flüssigkeit ist an der oder in der Verdampferkartusche bevorratet. Als Flüssigkeit kommen unterschiedliche Mischungen mit verschiedenen Bestandteilen gleicher oder unterschiedlicher Dampfdichten zum Einsatz. Eine typische Mischung für den Einsatz in einer E-Zigarette weist z.B. Bestandteile von Glycerin und Propylenglycol auf, ggf. angereichert um Nikotin und/oder nahezu beliebige Geschmacksstoffe. Für den Einsatz im medizinischen oder therapeutischen Bereich, z.B. zur Inhalation von Asthma-Präparaten, kann die Mischung entsprechend medizinische Bestandteile und Wirkstoffe aufweisen.

Die einzelnen Bestandteile der Verdampferkartusche, nämlich der Hohlkörper, der Vorratstank und die Verdampfereinheit, können in einem gemeinsamen Bauteil zusammengefasst sein, wobei dieses Bauteil dann ein Einwegartikel ist, der für eine endliche Anzahl von Inhalationszügen durch eine konsumierende Person ausgelegt ist und zusammen mit einem Kartuschenträger als wiederverwendbarem Mehrwegartikel, der mindestens eine elektronische Steuereinheit und eine Energiequelle umfasst, einen Inhalator bildet. Die Verdampferkartusche kann jedoch auch erst durch das Zusammenfügen mehrerer Bauteile gebildet sein, wobei einzelne Bauteile, nämlich insbesondere der Hohlkörper und die Verdampfereinheit, in dem Kartuschenträger als Mehrwegartikel angeordnet sind, und der Vorratstank als separates Bauteil den Einwegartikel bildet. Letztlich lässt sich der Inhalator durch Austausch des Einwegartikels, der üblicherweise die Flüssigkeit beinhaltet, variabel einsetzen.

Entsprechend sind der Einwegartikel und der Mehrwegartikel lösbar miteinander verbunden. Der Kartuschenträger als Mehrwegartikel umfasst üblicherweise mindestens eine elektronische Steuereinheit und eine Energiequelle. Die Energiequelle kann z.B. eine elektrochemische Einwegbatterie oder ein wiederaufladbarer elektrochemischer Akku, z.B. ein Li-lonen-Akku sein, mittels dem das Heizorgan über elektrische Kontakte der Verdampfereinheit mit Energie versorgt wird. Die elektronische und/oder elektrische Steuereinheit dient zum Steuern der Verdampfereinheit innerhalb der Verdampferkartusche. Der Kartuschenträger kann aber auch Bestandteile der Verdampferkartusche umfassen. Der Einwegartikel kann als Ansteckteil an den Mehrwegartikel ansteckbar oder als Einsetzteil in den Mehrwegartikel einsetzbar ausgebildet sein. Anstelle einer Steckverbindung sind auch Schraubverbindungen, Schnappverbindungen oder andere Schnellverbindungen einsetzbar. Mit der Verbindung von Einwegartikel und Mehrwegartikel wird eine mechanische und elektrische Kopplung zur Bildung eines funktionsbereiten Inhalators hergestellt.

Die zentrale und letztlich die Nutzung (z.B. als E-Zigarette oder als medizinischer Inhalator) bestimmende Komponente ist der Vorratstank als Bestandteil der Verdampferkartusche. Diese beinhaltet in der Regel die von der Person gewählte, gewünschte und/oder benötigte Flüssigkeit bzw. ein Flüssigkeitsgemisch (im Folgenden auch allgemein als Fluid bezeichnet) sowie den den Strömungskanal bildenden Hohlkörper und die Verdampfereinheit. Das Fluid ist in dem Vorratstank der Verdampferkartusche bevorratet. Mittels der flüssigkeitspermeablen Verdampfereinheit wird das Fluid aus dem Vorratstank aufgrund zumindest initial kapillarer Förderung durch das Dochtorgan und das Heizorgan geleitet. Die von einer Energiequelle erzeugte Spannung, die an dem Heizorgan angelegt wird, führt zu einem Stromfluss im Heizorgan. Aufgrund des Heizwiderstandes, vorzugsweise des Ohm'schen Widerstands des Heizorgans führt der Stromfluss zu einer Erhitzung des Heizorgans und letztlich zu einer Verdampfung des in der Verdampfereinheit befindlichen Fluids. Der auf diese Weise erzeugte Dampf und/oder Aerosol entweicht aus der Verdampfereinheit in Richtung des Strömungskanals und wird als Dampfzugabe der Luftströmung beigemischt. Das Fluid hat damit einen vorgegebenen Weg mit einer vorgegebenen Strömungsrichtung, nämlich als Fluid durch das Dochtorgan an das und durch das Heizorgan und gasförmig aus dem Heizorgan in den Strömungskanal. In dem Strömungskanal wird das verdampfte Fluid durch den Luftstrom mitgerissen, wobei sich Dampf/Nebel und/oder Aerosol bildet, wenn der Strömungskanal mit einem Druck/Unterdruck beaufschlagt wird, indem z.B. eine konsumierende Person an dem Strömungskanal saugt oder eine Pumpe einen Luftstrom durch den Strömungskanal fördert.

Damit das Fluid aus dem Vorratstank nicht direkt in den Strömungskanal fließt, deckt die Verdampfereinheit den Zugang vom Vorratstank zum Strömungskanal vollständig ab. Vollständig abgedeckt bedeutet in diesem Zusammenhang, dass die Flüssigkeit zwingend durch die Verdampfereinheit geführt ist, so dass das Fluid nicht direkt vom Vorratstank in den Strömungskanal gelangen kann, sondern den "Umweg" über das Dochtorgan und das Heizorgan nehmen muss. Das Dochtorgan dient zum einen zum Zwischenspeichern von Fluid, um insbesondere bei nahezu entleertem Vorratstank noch ausreichend Fluid für wenige Züge am Inhalator zur Verfügung zu stellen. Das Dochtorgan dient zum anderen insbesondere zum Transport des Fluids vom Vorratstank in Richtung des Strömungskanals und wirkt gleichzeitig als eine Art Rückschlagschutz, um den Rücklauf von Fluid und/oder Gas bzw. Dampf in Richtung des Vorratstanks zu unterbinden sowie eine Anreicherung einzelner Bestandteile des Fluids bei höheren Temperaturen zu verhindern.

Bisher bekannte Verdampferkartuschen weisen eine Verdampfereinheit mit einem Dochtorgan auf, der aus mehreren miteinander verwobenen/verdrillten Fäden/Fasern z.B. aus Baumwolle oder aus Glasfasern gebildet ist. Dieser Faserdocht weist kapillare Eigenschaften auf, die dazu führen, dass beim initialen Kontakt mit dem Fluid, der Faserdocht taucht in den Vorratstank ein, das Fluid in dem Vorratstank aufgenommen und in Richtung des Heizorgans gefördert wird. Das Heizorgan ist üblicherweise in Form einer Glühwendel ausgebildet. Dieser gewickelte Metalldraht besteht beispielsweise aus Edelstahl, Kupfer, Kupferverbindungen oder Nickel. Diese Verdampfereinheit ist in der Regel nur manuell herzustellen und weist eine begrenzte Speicherkapazität zum Zwischenspeichern von Fluid auf. Ein weiterer Nachteil besteht in der geringen Transportrate von Fluid aufgrund der begrenzten Anzahl von Mikrokanälen. Außerdem ergibt sich eine inhomogene und nicht regelbare Temperaturverteilung längs des Docht-Wendel-Systems. Mit anderen Worten ist eine gleichmäßige und kontinuierliche Versorgung des Heizorgans mit dem Fluid nur begrenzt sichergestellt. Außerdem besteht die Gefahr lokaler Überhitzung mit der Folge von Schadstoffentstehung. Des Weiteren weist diese Lösung keinen Rückschlagschutz auf.

Bei anderen bekannten Lösungen umfasst die Verdampfereinheit daher einen einstückigen Dochtblock als Dochtorgan. Dieser üblicherweise aus keramischen Werkstoffen bestehende Dochtblock vereinfacht die automatisierte Herstellung der Verdampfereinheit und der Verdampferkartusche und weist mehrere Mikrokanäle für eine gegenüber dem Faserdocht erhöhte Transportrate auf. Dennoch weist auch diese Lösung mehrere Nachteile auf. Neben einer weiterhin limitierten Transportrate und Zwischenspeicherkapazität ist der Einsatz solcher blockartigen Dochtblöcke sehr unflexibel und vor allem schwierig zu montieren, da die Dochtblöcke nur in exakt - in einem engen Toleranzbereich - vorgefertigte Aufnahmen/Halterungen oder dergleichen einsetzbar sind.

Bei einem einstückigen Dochtkörper kann dieser selbst als Heizorgan dienen, wenn das z.B. keramische Material des Dochtkörpers, der Mikrokanäle aufweist, elektrisch leitfähig ausgebildet ist. Dann weist der Dochtkörper eine Doppelfunktion auf und bildet die Verdampfereinheit. In anderen Fällen kann zusätzlich zum Dochtkörper eine separate Komponente als Heizorgan dienen. In dem letztgenannten Fall bilden der Dochtkörper und das separate Heizorgan die Verdampfereinheit. Das Heizorgan ist dann üblicherweise ein flächiges und flaches, z.B. im Wesentlichen aus Silizium bestehendes oder Silizium oder p- oder n-dotiertes Silizium aufweisendes MEMS-Bauteil (Micro-electro-mechanical-system-Bauteil), das flüssigkeitspermeabel ausgebildet ist. Die zuvor genannten Nachteile verstärken sich insbesondere bei Lösungen, bei denen Dochtorgan und Heizorgan separate Körper sind, die in einem Kontaktbereich aneinanderliegen.

Ein weiterer Nachteil entsteht dadurch, dass im Kontaktbereich unterschiedliche Oberflächenrauigkeiten aufeinanderliegen, nämlich einerseits die poröse Struktur des Dochtorgans und andererseits die üblicherweise glatte Oberfläche des Heizorgans. In dem Kontaktbereich entstehen aufgrund der unterschiedlichen Rauigkeiten undefinierte und nicht Mikrokanäle bildende Hohlräume. Diese Hohlräume führen zu einer unzureichenden Fluidversorgung des Heizorgans mit dem Fluid. Anders ausgedrückt behindern solche Hohlräume eine ausreichende Fluidkopplung zwischen der Austrittsseite des Dochtorgans und der Eintrittsseite des Heizorgans. Weitere Hohlräume entstehen durch eine nicht planparallele Ausrichtung der Oberflächen von Dochtorgan und Heizorgan, z.B. durch gewölbte Oberflächen und/oder Montagefehler. Das führt zu einer unzureichenden Reproduzierbarkeit der Montageergebnisse, wodurch sich schwankende Verdampfungsbedingungen ergeben. Die bestehenden Hohlräume können zu einer thermisch isolierenden Dampfblasenbildung, dem so genannten Leidenfrosteffekt führen, mit dem unerwünschten Effekt einer (lokalen) Überhitzung. Im Übrigen behindern Dampfblasen das Nachfördern des Fluids aus dem Dochtorgan in das Heizorgan.

Bekannte Verdampferkartuschen mit den Merkmalen des Oberbegriffes des Anspruches 1 sind u.a. aus der EP 3 200 559 A2, US 2017/367411 A1, DE 10 2017 123870 A1, US 2016/316819 A1, GB 2 561 867 A, EP 3 417 726 A1, WO 2018/211035 A1 sowie der WO 2013/083638 A1 bekannt.

Die Erfindung ist im unabhängigen Anspruch 1 definiert. Die abhängigen Ansprüche beschreiben bevorzugte Ausbildungen der Erfindung.

Der Erfindung liegt somit die Aufgabe zugrunde, eine kompakte Verdampferkartusche vorzuschlagen, die konstante und reproduzierbare Verdampfungsbedingungen sicherstellt. Die Aufgabe besteht weiterhin darin, einen entsprechenden Inhalator zu schaffen.

Diese Aufgabe wird durch eine Verdampferkartusche der eingangs genannten Art dadurch gelöst, dass das Dochtorgan aus einer Vielzahl granulatartiger Körner gebildet ist, die aufgrund ihrer Schüttung und/oder Ausbildung Mikrokanäle bilden. Mit Schüttung ist sowohl das lose als auch das verbundene Nebeneinanderliegen der Körner beschrieben, wobei auch gerüttelte und/oder verdichtete Anordnungen der Körner umfasst sind. Mit Ausbildung der Körner ist z.B. beschrieben, dass die Körner selbst Mikrohohlräume und/oder Mikrokanäle aufweisen können. Somit werden in der Verdampfereinheit zwischen den einzelnen, aneinander liegenden Körnern und/oder durch einzelne Körner eine Vielzahl zufälliger Mikrokanäle zwischen dem Vorratstank und dem Strömungskanal gebildet, die eine konstante und gleichmäßige Verdampfung auf der Austrittsseite der Verdampfereinheit sicherstellen. Anders ausgedrückt wird durch das granulare Dochtorgan eine optimale Fluidkopplung zwischen der Eintrittsseite in die Verdampfereinheit und der Austrittsseite aus der Verdampfereinheit hergestellt. Mit der erfindungsgemäßen Ausbildung des Dochtorgans werden gegenüber den aus dem Stand der Technik bekannten Lösungen eine Fülle von Vorteilen erzielt. Neben der verbesserten Transportrate, es wird mehr und vor allem gleichmäßig Fluid aus dem Vorratstank durch die Verdampfereinheit geführt, gewährleistet das granulare Dochtorgan aufgrund der körnigen und somit porösen Dochtstruktur eine erhöhte Zwischenspeicherkapazität für Fluid. Des Weiteren verbessert ein granulares Dochtorgan den Rückschlagschutz, da die gebildeten Mikrokanäle einen nichtlinearen Verlauf aufweisen. Besonders vorteilhaft gestaltet sich die Montage eines granularen Dochtorgans, da sich dieses an dem jeweiligen Montageort an jede beliebige Kontur/Geometrie der Aufnahme des Dochtorgans anpassen kann. Durch die Kornstruktur passt sich das Dochtorgan beim Montieren/Abfüllen des granularen Materials flexibel an die jeweilige Kontur/Geometrie an und füllt - unter Bildung von Mikrokanälen und/oder Mikrohohlräumen - unerwünschte, nicht Mikrokanäle bildende Hohlräume auf und vermeidet Spaltbildung zu angrenzenden Flächen. Im Ergebnis werden durch das granulare Dochtorgan konstante und reproduzierbare Verdampfungsbedingungen sicherstellt. Dabei spielt es keine Rolle, ob die Verdampfereinheit - mit dem Dochtorgan und/oder dem Heizorgan als Bestandteil der Verdampferkartusche - an dem oder in dem Kartuschenträger angeordnet ist, also am/im Mehrwegartikel, oder ob die Verdampfereinheit am/im Einwegartikel angeordnet ist.

Erfindungsgemäß sind das Dochtorgan und das Heizorgan separate Einheiten, die in einem Kontaktbereich aneinander liegen, wobei das Dochtorgan dem Vorratstank und das Heizorgan dem Strömungskanal zugewandt ist, und das Heizorgan elektrische Kontakte zur elektrischen Kontaktierung mit der Energiequelle aufweist, und wobei das Dochtorgan Mikrokanäle aufweist und das Heizorgan, das vom Dochtorgan gegenüber dem Vorratstank abgeschirmt ist, flüssigkeits- und dampfpermeabel ausgebildet ist. Die Mikrokanäle im Dochtorgan sind in der zuvor beschriebenen Weise durch die Körneranordnung und/oder die Körner selbst gebildet. Die Durchlässigkeit des Heizorgans für Flüssigkeit und Gas bzw. Dampf kann z.B. durch Bohren, Lasern, Ätzen oder dergleichen gebildet sein. Bei dieser quasi "zweiteiligen" Ausbildung der Verdampfereinheit kann jedes Element für sich optimal an die jeweilige Funktionalität angepasst sein, nämlich das Dochtorgan insbesondere an das Speichern und Leiten der Flüssigkeit aus dem Vorratstank an das Heizorgan, und das Heizorgan insbesondere an das Übernehmen der Flüssigkeit aus dem Dochtorgan und das Umwandeln der Flüssigkeit in Gas bzw. Dampf sowie Abgeben desselben in den Strömungskanal.

Erfindungsgemäß ist die minimale Korngröße der Körner des Dochtorgans mindestens im Kontaktbereich zum Heizorgan größer als der mittlere Durchmesser der Durchgänge des Heizorgans. Dadurch wird ein Verstopfen der Durchgänge des Heizorgans wirksam verhindert.

Die Mikrokanäle des Dochtorgans, unabhängig davon, ob es nur die Dochtfunktion oder eine kombinierte Docht- und Heizfunktion aufweist, erstrecken sich durchgängig von einer Eintrittsseite E_{D} zu einer Austrittsseite A_{D}, so dass Fluid entweder vom Vorratstank durch das Dochtorgan in Richtung des Strömungskanals transportiert und dabei erhitzt wird, so dass gasförmiges Liquid/Dampf aus dem Dochtorgan in den Strömungskanal transportiert wird, oder Fluid vom Vorratstank zum Heizorgan transportiert wird, das flüssigkeits- und dampfpermeabel ist und aus dem Fluid Dampf erzeugt, der aus dem Heizorgan in den Strömungskanal transportiert wird.

Die Verdampferkartusche kann einerseits dadurch gebildet sein, dass ein Vorratstank als Bestandteil der Verdampferkartusche als Einwegartikel mit mindestens einem Kartuschenträger als Mehrwegartikel, der neben einer Steuereinheit und einer Energiequelle z.B. die Verdampfereinheit als Bestandteil der Verdampferkartusche umfasst, verbunden wird. Andererseits kann die Verdampferkartusche eine mindestens aus Verdampfereinheit und Vorratstank gebildete Einheit als Einwegartikel sein, die zum mechanischen und elektrischen Verbinden mit mindestens einem mindestens eine elektronische Steuereinheit und eine Energiequelle umfassenden Kartuschenträger als Mehrwegartikel zur Bildung eines Inhalators ausgebildet und eingerichtet ist, wobei die Verdampfereinheit elektrische Kontakte zur elektrischen Kontaktierung mit der Energiequelle umfasst. Dadurch sind kompakte Verdampferkartuschen/Inhalatoren mit konstanten und reproduzierbaren Verdampfungsbedingungen geschaffen.

Eine zweckmäßige Weiterbildung ist dadurch gekennzeichnet, dass das das granulare Dochtorgan bildende, körnige Material mindestens teilweise elektrisch leitfähig ist. Damit bildet das Dochtorgan gleichzeitig auch noch das Heizorgan. Die Verdampfereinheit ist entsprechend ein einzelnes Organ, also quasi "einteilig" ausgebildet, wodurch eine besonders kompakte Bauweise erreicht wird. Die Fluidkopplung zwischen Dochtorgan und Heizorgan ist bei dieser Variante optimal, da die Mikrokanäle unterbrechungsfrei und durchgängig von der Eintrittsseite in die Verdampfereinheit bis zur Austrittsseite aus der Verdampfereinheit ausgebildet sind.

In einer bevorzugten Ausführungsform ist im Bereich der Zugangsöffnung ein Aufnahmeraum zum Aufnehmen der Verdampfereinheit ausgebildet, wobei der Aufnahmeraum zum Aufnehmen von Flüssigkeit aus dem Vorratstank in die Verdampfereinheit und zum Abgeben von gasförmigem Liquid/Dampf aus der Verdampfereinheit in den Strömungskanal mindestens teilweise durch eine flüssigkeits- und gas- bzw. dampfpermeable Struktur begrenzt ist. Der Aufnahmeraum für die Verdampfereinheit kann durch Gehäusewände, Vorsprünge, Platten, Deckel, Wickelelemente, Schieber oder jede andere Begrenzung oder Kombinationen davon gebildet sein. Entscheidend ist, dass mindestens ein flüssigkeits- und dampfpermeabler Zugang aus dem Vorratstank und mindestens ein flüssigkeits- und gas- bzw. dampfpermeabler Ausgang zum Strömungskanal existiert. Der Aufnahmeraum hält das Dochtorgan und das Heizorgan zusammen und in Position, und zwar vor der Zugangsöffnung zwischen Vorratstank und Strömungskanal, so dass die Flüssigkeit einerseits zuverlässig darin gehindert wird, direkt aus dem Vorratstank in den Strömungskanal zu fließen, und andererseits gleichmäßig und konstant in Richtung Strömungskanal geführt wird. Durch die Fläche des Ausgangs zum Strömungskanal und des Zugangs vom Vorratstank kann der Partialdruck über dem Dochtorgan eingestellt werden

In einer vorteilhaften Weiterbildung umfasst die Verdampferkartusche ein Trägerelement, das den Hohlkörper bildet und zum einen eine Durchgangsöffnung zur Bildung des Strömungskanals aufweist, und zum anderen eine Ausnehmung zur Aufnahme der Verdampfereinheit aufweist. Mit dieser Ausbildung ist eine besonders kompakte Verdampferkartusche geschaffen.

Vorteilhafterweise ist ein aus dem granularen und elektrisch leitfähigen Material gebildetes Sediment fixiert, derart, dass das Sediment eine dreidimensionale Widerstandsheizmatrix mit parallelen und/oder in Reihe geschalteten Widerständen bildet. Damit ist das Dochtorgan neben der Dochtfunktion des kapillaren Fluidtransports besonders effektiv auch als Heizorgan für definierte und reproduzierbare Verdampfungsbedingungen einsetzbar. Das elektrisch leitende Dochtorgan kann beispielsweise über Presskontakte elektrisch angebunden werden, wobei mindestens zwei Kontakte benötigt werden.

Vorzugsweise sind die Körner des Dochtorgans hinsichtlich ihrer Materialauswahl und/oder ihrer Größe gleich und/oder ungleich ausgebildet. Das bedeutet, dass Körner innerhalb eines Dochtorgans alle aus dem gleichen Material und alle von gleicher Größe (gemeint ist eine Größenordnung innerhalb eines definierten Bereiches) sind, oder alle Körner aus einem Material aber unterschiedlicher Größe sind, oder die Körner aus unterschiedlichen Materialien aber alle von gleicher Größe sind, oder die Körner aus unterschiedlichen Materialien und unterschiedlicher Größe sind. Damit sind Dochtorgane z.B. für unterschiedliche Flüssigkeiten und Flüssigkeitsmischungen z.B. mit unterschiedlichen Transportraten (Flüssigkeitsversorgung) und/oder unterschiedlichen Wärmeleitfähigkeiten und/oder unterschiedlichen Strömungswiderständen (z.B. für einen angepassten Rückschlagschutz) auf einfache Weise individuell zusammenstellbar.

Eine bevorzugte Ausführungsform ist dadurch gekennzeichnet, dass die Körner des Dochtorgans ausgehend vom Vorratstank in Richtung des Strömungskanals lokal unterschiedliche Korngrößen aufweisen. Beispielsweise können die Körner schichtweise hinsichtlich ihrer Größe variieren. Dieser schichtweise Aufbau kann noch flexibel bei der Montage/Abfüllung erfolgen, um z.B. den Strömungsgradienten in dem granularen Dochtorgan einzustellen.

Vorteilhafterweise bestehen die Körner des Dochtorgans ausgehend vom Vorratstank in Richtung des Strömungskanals lokal aus unterschiedlichen Materialien. Mit der schichtweisen Veränderung des Materials der Körner kann z.B. eine veränderliche Wärmeleitfähigkeit des Dochtorgans erreicht werden, indem z.B. auf der dem Vorratstank zugewandten Seite des Dochtorgans Materialien mit einer geringen Wärmeleitfähigkeit eingesetzt werden, um z.B. eine Wärmeübertragung auf die im Vorratstank befindliche Flüssigkeit zu vermeiden, während auf der dem Strömungskanal zugewandten Seite des Dochtorgans Materialien mit einer hohen Wärmeleitfähigkeit eingesetzt werden, um den Verdampfungsprozess in Richtung des Strömungskanals zu unterstützen bzw. die Wärme im Übergangsbereich zum Strömungskanal zu halten.

Vorzugsweise liegen die Körner des Dochtorgans als lose Schüttung innerhalb des Aufnahmeraums. Bei dieser Variante ist eine besonders gleichmäßige und vor allem hohlraum- und spaltreduzierte Verteilung der Körner innerhalb des Aufnahmeraums gewährleistet, wodurch konstante und reproduzierbare Verdampfungsbedingungen weiter optimiert sind.

In einer anderen vorteilhaften Variante liegen die Körner des Dochtorgans als miteinander verbundene Schüttung innerhalb des Aufnahmeraums. Die Verbindung der geschütteten Körner, z.B. mittels pastöser Komponenten oder dergleichen, stellt sicher, dass eine definierte Struktur geschaffen ist, die ebenfalls konstante und reproduzierbare Verdampfungsbedingungen weiter optimiert und insbesondere für den Fall einer elektrischen Leitfähigkeit der Körner ein fixiertes Sediment bereitstellt.

Die bevorzugte Korngröße beträgt zwischen 0,1µm und 2mm, besonders bevorzugt zwischen 3µm und 300µm. Mit einer Korngröße innerhalb dieser Bereiche sind die zuvor geschilderten Vorteile besonders effektiv erreichbar.

Zweckmäßigerweise liegt die maximale Korngröße in Abhängigkeit der Fließeigenschaft der jeweils zu fördernden Flüssigkeit außerhalb einer die kapillare Förderung ausschließenden Größe. Mit anderen Worten wird durch die Begrenzung der maximalen Korngröße das Bilden von Mikrokanälen innerhalb des Dochtorgans sichergestellt, um insbesondere den - zumindest initial - kapillaren Förderstrom der Flüssigkeit für eine gleichmäßige und konstante Versorgung zu gewährleisten.

Vorteilhafterweise bestehen die Körner des Dochtkörpers aus Sand und/oder Graphit. Diese kostengünstigen Kornarten stehen in der Natur nahezu grenzenlos zur Verfügung und sind chemisch inert und umweltverträglich.

In einer bevorzugten Weiterbildung sind die Körner des Dochtorgans mindestens teilweise magnetisch. Dadurch lassen sich die Körner gezielt ausrichten, um z.B. den Strömungswiderstand zu beeinflussen.

Dadurch, dass das Dochtorgan aus granulatartigen Körnern gebildet ist, liegt das Dochtorgan eng und flächig am Heizorgan an. Es ist also eine gleichmäßige Anlage des Dochtorgans am Heizorgan gewährleistet, wodurch eine optimierte Fluidkopplung einerseits und eine homogene thermische Anbindung andererseits geschaffen ist. In montiertem/geschüttetem Zustand liegt das Dochtorgan mit einer Eintrittsseite in Richtung des Vorratstanks in der Flüssigkeit und mit der Austrittsseite am Heizorgan an. Das Heizorgan wiederum liegt mit einer Eintrittsseite am Dochtorgan an und weist mit einer Austrittsseite in den Strömungskanal. Mit der bevorzugten Ausführungsform sind eine konstante und homogene Liquidversorgung des Heizorgans und damit reproduzierbare Verdampfungsbedingungen sichergestellt.

Vorteilhafterweise ist das Heizorgan ein im Wesentlichen aus Silizium bestehendes oder Silizium oder p- oder n-dotiertes Silizium aufweisendes MEMS-Bauteil (Micro-electro-mechanical-system), das ausgehend von einer dem Dochtorgan zugewandten Oberseite bis zu einer dem Strömungskanal zugewandten Unterseite flüssigkeits- und dampfpermeable Durchgänge aufweist. Mit diesem platzsparenden Heizorgan ist eine besonders effektive Dampfbildung erzielbar.

In einer bevorzugten Ausführungsform umfasst die Verdampferkartusche ein Gehäuse, das den Hohlkörper und die Verdampfereinheit umgibt, wobei die Gehäusewand den Vorratstank zur Umgebung hin abgrenzt. Damit ist eine einfache und bauraumsparende Bauweise gewährleistet.

In einer zweckmäßigen Variante hält die Gehäusewand das Dochtorgan in seiner Position. Durch die Doppelfunktion der Gehäusewand als Begrenzung für den Vorratstank und als Fixiermittel des Dochtorgans innerhalb des Aufnahmeraums ist eine einfache Bauweise geschaffen.

Die Aufgabe wird auch durch einen Inhalator der eingangs gennannten Art dadurch gelöst, dass die Verdampferkartusche nach einem oder mehreren der Ansprüche 1 bis 19 ausgebildet und eingerichtet ist.

Die sich daraus ergebenden Vorteile wurden bereits im Zusammenhang mit der Verdampferkartusche beschrieben, weshalb zur Vermeidung von Wiederholungen auf die vorstehenden Ausführungen verwiesen wird.

Weitere zweckmäßige und/oder vorteilhafte Merkmale und Weiterbildungen zur Verdampferkartusche und dem Inhalator ergeben sich aus den Unteransprüchen und der Beschreibung. Besonders bevorzugte Ausführungsformen der Verdampferkartusche und des Inhalators werden anhand der beigefügten Zeichnung näher erläutert. In der Zeichnung zeigt:
- Fig. 1: eine schematische Darstellung einer bevorzugten Ausführungsform eines erfindungsgemäßen Inhalators mit Kartuschenträger und Verdampferkartusche im Teilschnitt,
- Fig. 2: eine vergrößerte Darstellung einer weiteren Ausführungsform einer Verdampferkartusche im Teilschnitt,
- Fig. 3: eine vergrößerte Darstellung einer bevorzugten Ausführungsform eines Teils einer Verdampferkartusche im Schnitt,
- Fig. 4: eine weitere Ausführungsform eines Teils einer Verdampferkartusche,
- Fig. 5: eine weitere Ausführungsform eines Teils einer Verdampferkartusche,
- Fig. 6: eine vergrößerte Darstellung der Ausführungsform der Verdampferkartusche gemäß Figur 1 im Teilschnitt,
- Fig. 7: eine vergrößerte Darstellung einer weiteren Ausführungsform eines Teils einer Verdampferkartusche,
- Fig. 8: die Verdampferkartusche gemäß Figur 7 entlang des Schnitts A-A,
- Fig. 9: eine vergrößerte Darstellung einer weiteren Ausführungsform einer Verdampferkartusche im Teilschnitt,
- Fig. 10: die Verdampferkartusche gemäß Figur 9,
- Fig. 11: eine vergrößerte Darstellung einer weiteren Ausführungsform einer Verdampferkartusche im Teilschnitt, und
- Fig. 12: die Verdampferkartusche gemäß Figur 11.

Die in der Zeichnung dargestellte Verdampferkartusche sowie der Inhalator dienen zum Inhalieren von mit Wirkstoffen, z.B. Nikotin, angereichertem Dampf und/oder Aerosolen aus Flüssigkeiten, sind entsprechend im Zusammenhang mit einer E-Zigarette beschrieben. Die Verdampferkartusche und der Inhalator sind in gleicher Weise zum Inhalieren von mit medizinischen Wirkstoffen angereichertem Dampf aus pharmazeutischen und/oder nahrungsergänzenden Produkten einsetzbar.

Die dargestellte Verdampferkartusche 10 umfasst einen Hohlkörper 15 mit einem durchgängigen Strömungskanal 16 sowie einen Vorratstank 17 zum Bevorraten von Flüssigkeit, wobei der Vorratstank 17 mindestens eine Zugangsöffnung 18 zum Strömungskanal 16 aufweist und im Bereich jeder Zugangsöffnung 18 eine sich über die gesamte Zugangsöffnung 18 erstreckende Verdampfereinheit 19 angeordnet ist, die ein Dochtorgan 20 und ein Heizorgan 21 aufweist, wobei die Verdampfereinheit 19 flüssigkeitspermeabel ausgebildet ist, derart, dass Flüssigkeit mindestens initial kapillar aus dem Vorratstank 17 durch die Verdampfereinheit 19 in Richtung des Strömungskanals 16 förderbar ist.

Der Hohlkörper 15 mit seinem mindestens einen Strömungskanal 16, zwei oder mehr Strömungskanäle 16 können ebenfalls vorgesehen sein, bildet einen Saugkanal/Schlot. Die Form des Hohlkörpers 15 kann ebenso wie der Verlauf des Strömungskanals 16 nahezu beliebig sein. Entscheidend ist, dass die Eintrittsseite Es jedes Strömungskanals 16 offen zur Umgebung ist, um z.B. Luft ansaugen zu können, und dass die Austrittsseite As offen ist, um z.B. einen Unterdruck erzeugen zu können, insbesondere durch das Saugen einer konsumierenden Person. Offen bedeutet in diesem Zusammenhang, dass die Eintrittsseite Es und die Austrittsseite As luftdurchlässig sind. Im Bereich der Zugangsöffnung 18 zwischen Vorratstank 17 und Strömungskanal 16 bildet die Verdampfereinheit 19 eine Art Flüssigkeitssperre, die verhindert, dass Flüssigkeit aus dem Vorratstank 17 direkt und als Flüssigkeit in den Strömungskanal 16 fließt. Unabhängig von der Form und Ausbildung des Vorratstanks 17, es können auch zwei oder mehr Vorratstanks 17 vorgesehen sein, und des Hohlkörpers 15 sowie der Anordnung/Positionierung von Vorratstank 17 zu Hohlkörper 15 stellt die Verdampfereinheit 19 sicher, dass Flüssigkeit zwangsläufig aus dem Vorratstank 17 in Richtung des Strömungskanals 16 geführt wird und spätestens beim Austritt aus der Verdampfereinheit 19 als Gas bzw. Dampf in den Strömungskanal 16 abgegeben wird.

Diese Verdampferkartusche 10 zeichnet sich erfindungsgemäß dadurch aus, dass das Dochtorgan 20 aus einer Vielzahl granulatartiger Körner 24 gebildet ist, die aufgrund ihrer Schüttung und/oder Ausbildung Mikrokanäle 23 bilden. Die aneinander liegenden Körner 24 bilden zum einen mit benachbarten Körnern 24 Mikrohohlräume, zum anderen können die Körner 24 selbst Mikrohohlräume, so genannte Poren, aufweisen. Durch die Anbindung und das Zusammenwirken sämtlicher Mikrohohlräume in den und zwischen den Körnern 24 sind die Mikrokanäle 23 gebildet, die eine mindestens initial kapillare Förderung gewährleisten sowie durchgängig ausgebildet sind und einen nichtlinearen Verlauf aufweisen. Beim Durchtritt durch die das Dochtorgan 20 und das Heizorgan 21 umfassenden Verdampfereinheit 19 wird im Betrieb der Verdampferkartusche 10 aus der Flüssigkeit des Vorratstanks 17 zum Strömungskanal 16 hin Dampf und/oder Aerosol gebildet, wobei die poröse Struktur des Dochtorgans 20 einerseits ein Speichermedium für Flüssigkeit bildet und andererseits einen Strömungswiderstand darstellt. Die Strömungsrichtung der Flüssigkeit erfolgt vom Vorratstank 17 durch die Verdampfereinheit 19 in Richtung des Strömungskanals 16.

Die erfindungsgemäße Verdampferkartusche 10 kann als Einwegartikel eine bauliche Einheit sein, die die Komponenten Hohlkörper 15, Vorratstank 17 und Verdampfereinheit 19 beinhaltet. Die Verdampferkartusche 10 kann aber auch mehrteilig ausgebildet sein, wobei sich Komponenten der Verdampferkartusche 10 auf den Einwegartikel und den Mehrwegartikel verteilen, derart, dass z.B. der Vorratstank 17 ein Einwegartikel ist, der erst beim Zusammenführen mit einem Kartuschenträger 13, der ein Mehrwegartikel sein kann und neben einer elektronischen Steuereinheit 11 und einer Energiequelle 12 auch Bestandteile der Verdampferkartusche 10, wie z.B. den Hohlkörper 15 und die Verdampfereinheit 19, umfassen kann, zur baulichen Einheit der Verdampferkartusche 10 führt. Die Verdampferkartusche 10 definiert sich entsprechend über ihre Komponenten, nämlich Hohlkörper 15 mit Strömungskanal 16, Vorratstank 17 und Verdampfereinheit 19, und nicht über die konstruktive/bauliche Zuordnung der Komponenten zum Mehrwegartikel bzw. Einwegartikel.

Die im Folgenden beschriebenen Merkmale und Weiterbildungen stellen für sich betrachtet oder in Kombination miteinander bevorzugte Ausführungsformen dar. Es wird ausdrücklich darauf hingewiesen, dass Merkmale, die in den Ansprüchen und/oder der Beschreibung und/oder der Zeichnung zusammengefasst oder in einer gemeinsamen Ausführungsform beschrieben sind, auch funktional eigenständig die weiter oben beschriebene Verdampferkartusche 10 weiterbilden können.

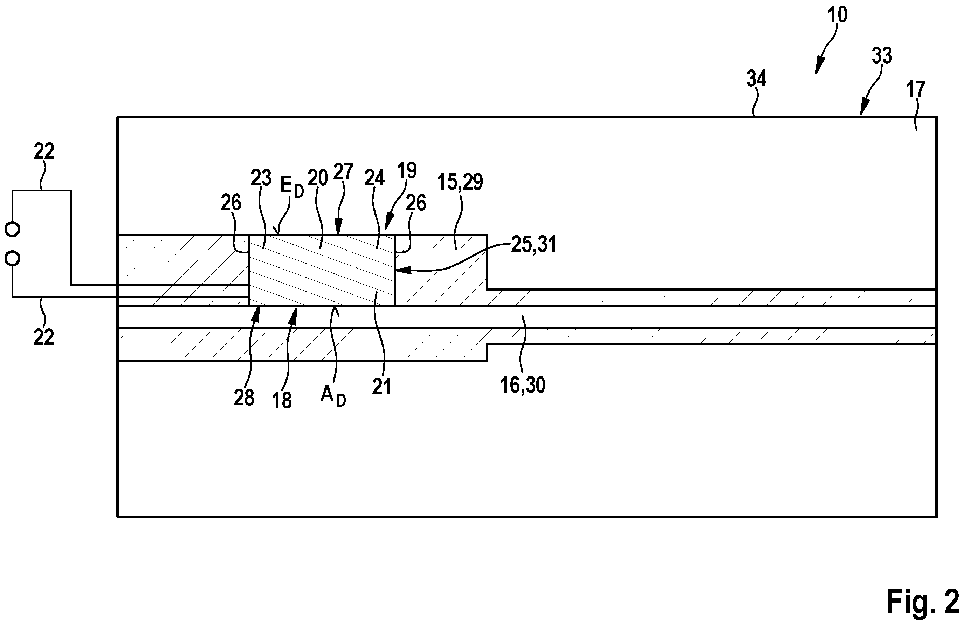

Das Dochtorgan 20 weist Mikrokanäle 23 auf, die sich durchgängig von einer Eintrittsseite E_{D} des Dochtorgans 20 zu einer Austrittsseite A_{D} des Dochtorgans 20 erstrecken. In einer ersten Ausführungsform bildet das Dochtorgan 20 gleichzeitig das Heizorgan 21 (siehe insbesondere Figur 2). Das Dochtorgan 20 bildet somit die gesamte Verdampfereinheit 19. Dazu ist das das granulare Dochtorgan 20 bildende, körnige Material mindestens teilweise elektrisch leitfähig. Die elektrisch leitfähigen Körner 24 befinden sich vorzugsweise mindestens in einem unteren, dem Strömungskanal 16 zugewandten Bereich. Entsprechend sind die elektrischen Kontakte 22 in dem elektrisch leitfähigen Bereich des Dochtorgans 20 angeordnet. In dieser Ausführung erstrecken sich die Mikrokanäle 23 von der Eintrittsseite E_{D} des Dochtorgans 20, die der Flüssigkeit in dem Vorratstank 17 ausgesetzt ist, bis zur Austrittsseite A_{D} des Dochtorgans 20, die unmittelbar an den Strömungskanal 16 grenzt.

Bevorzugt ist die Verdampferkartusche 10 zum mechanischen und elektrischen Verbinden mit dem mindestens die elektronische Steuereinheit 11 und die Energiequelle 12 umfassenden Kartuschenträger 13 zur Bildung eines Inhalators 14 ausgebildet und eingerichtet, wobei die Verdampfereinheit 19 elektrische Kontakte 22 zur elektrischen Kontaktierung mit der Energiequelle 12 umfasst. Der Inhalator 14 kann z.B. durch eine inhalierende Person aktiviert werden, beispielsweise als E-Zigarette, oder z.B. durch eine Pumpe aktiviert werden, z.B. als medizinisches Instrument für den Fall, das die Person selbst nicht mehr oder nicht ausreichend saugen kann.

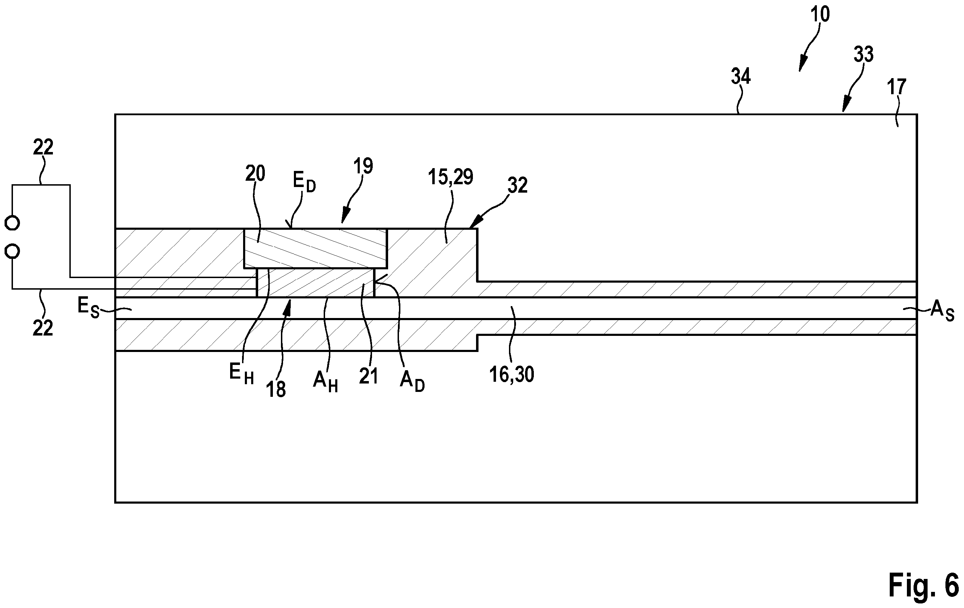

Im Bereich der Zugangsöffnung 18 ist ein Aufnahmeraum 25 zum Aufnehmen der Verdampfereinheit 19, im Beispiel der Figur 2 des Dochtorgans 20 und im Beispiel der Figur 6 des Dochtorgans 20 und des Heizorganes 21, ausgebildet, wobei der Aufnahmeraum 25 zum Aufnehmen von Flüssigkeit aus dem Vorratstank 17 in die Verdampfereinheit 19 und zum Abgeben von Dampf aus der Verdampfereinheit 19 in den Strömungskanal 16 mindestens teilweise durch eine flüssigkeits- und gas- bzw. dampfpermeable Struktur begrenzt ist. Der Aufnahmeraum 25 für die Verdampfereinheit 19 kann durch Gehäusewände anderer Komponenten der Verdampferkartusche 10, durch separate Wandelemente, Vorsprünge, Platten, Deckel, Wickelelemente, Schieber oder jede andere Begrenzung oder Kombinationen davon gebildet sein. Der Aufnahmeraum 25 kann jede beliebige Form und/oder Kontur aufweisen. Im Beispiel der Figuren 2 und 6 ist der Aufnahmeraum 25 z.B. umlaufend durch Wandelemente 26 begrenzt. Zum Vorratstank 17 gerichtet ist der Aufnahmeraum 25 mit der darin befindlichen Verdampfereinheit 19 beispielsweise durch ein Deckelelement 27, das flüssigkeitspermeabel ausgebildet ist, abgedeckt. Zum Strömungskanal 16 hin kann der Aufnahmeraum 25 z.B. durch eine flüssigkeits- und gas- bzw. dampfpermeable Gitterstruktur 28 (siehe Figur 2) oder durch ein separates Heizorgan 21 (siehe Figur 6), das entsprechend flüssigkeits- und dampfpermeable ausgebildet ist, begrenzt sein.

Vorzugsweise umfasst die Verdampferkartusche 10 ein Trägerelement 29, das den Hohlkörper 15 bildet und zum einen eine Durchgangsöffnung 30 zur Bildung des Strömungskanals 16 aufweist und zum anderen eine Ausnehmung 31 zur Aufnahme der Verdampfereinheit 19 aufweist. Das Trägerelement 29 ist vorzugsweise ein rohrförmiger Körper. In der Umfangswand des Körpers ist die Ausnehmung 31 ausgebildet, in der die Verdampfereinheit 19 angeordnet ist. Vorzugsweise ist die aus Trägerelement 29 und Verdampfereinheit 19 gebildete Einheit innerhalb eines den Vorratstank 17 bildenden Gehäuses 33 angeordnet, wobei das Innenvolumen des Vorratstanks 17 zwischen einer Gehäusewand 34 des Gehäuses 33 und dem Trägerelement 29 gebildet ist. Das Trägerelement 29 kann sich nur teilweise durch das Gehäuse 33 erstrecken (siehe z.B. Figur 2). In anderen Ausführungsformen kann sich das Trägerelement 29 auch vollständig (siehe z.B. Figur 6) durch das Gehäuse 33 erstrecken.

Die Körner 24 können als lose Schüttung innerhalb des Aufnahmeraums 25 bzw. in der Ausnehmung 31 liegen. In diesem Fall können sich die Körner 24 selbst im Betriebszustand der Verdampferkartusche 10 noch relativ zueinander bewegen und so variable Mikrokanäle 23 bilden. Die nebeneinander und übereinander liegenden Körner 24 stützen sich gegeneinander ab. Dabei können die Körner 24 rein mechanisch aneinanderstoßen. Die Körner 24 können aber auch untereinander mechanisch miteinander verzahnt sein. Entscheidend für die Bildung der Mikrokanäle ist entsprechend, dass die Körner 24 aneinanderliegen. In anderen Ausführungsformen, insbesondere in solchen, in denen das Dochtorgan 20 gleichzeitig als Heizorgan 21 ausgebildet ist, sind die Körner 24 innerhalb des Aufnahmeraums 25 bzw. in der Ausnehmung 31 mindestens teilweise miteinander verbunden. Besonders bevorzugt ist ein aus dem granularen und elektrisch leitfähigen Material gebildetes Sediment fixiert, derart, dass das Sediment eine dreidimensionale Widerstandsheizmatrix mit parallelen und/oder in Reihe geschalteten Widerständen bildet. An dieser Widerstandsheizmatrix sind die elektrischen Kontakte 22 ausgebildet bzw. angeordnet. Alle Mittel zum Fixieren der Körner 24 stellen dabei eine Flüssigkeitskopplung zwischen dem Vorratstank 17 und dem Heizorgan 21, wahlweise als Bestandteil des Dochtorgans 20 oder als separates Teil, sicher.

Die Körner 24 eines Dochtorgans 20 können hinsichtlich ihrer Materialauswahl und/oder ihrer Größe gleich und/oder ungleich ausgebildet sein. Alle Körner 24 können die gleiche Größe aufweisen, also in einem Größenbereich liegen. Die Körner 24 können jedoch unterschiedliche Größen aufweisen, also in unterschiedlichen Größenbereichen liegen. Bevorzugt beträgt die Korngröße zwischen 0,1 µm und 2mm und besonders bevorzugt zwischen 3µm und 300µm. Rein beispielhaft können alle Körner 24 in einem Größenbereich zwischen 50µm und 100µm (entspricht einem Größenbereich) liegen. Die Körner 24 eines Dochtorgans 20 können aber auch ausgehend vom Vorratstank 17 in Richtung des Strömungskanals 16 lokal unterschiedliche Korngrößen aufweisen. So können dem Vorratstank 17 nahestehende Schichten des Dochtorgans 20 Körner 24 mit einer Korngröße von z.B. 200µm bis 300µm (entspricht einem Größenbereich) aufweisen, während dem Strömungskanal 16 nahestehende Schichten des Dochtorgans 20 Körner 24 mit einer Korngröße von z.B. 50µm bis 100µm (entspricht einem Größenbereich) aufweisen. Durch die Wahl der Korngrößen und der jeweiligen Verteilung z.B. in Schichten mit Körnern 24 unterschiedlicher Größenbereiche lässt sich u.a. der Strömungswiderstand des Dochtorgans 20 individuell, letztlich sogar auch erst bei der Schüttung, einstellen. Durch die Auswahl der eingesetzten Korngrößen in einem Dochtorgan 20 lässt sich ein individueller Porengradient für das Dochtorgan 20 einstellen. Der minimale Korndurchmesser der Körner 24 im feinporigen Bereich sollte dabei vorzugsweise größer sein, als die Poren im nächst gröberen Bereich, um den Porengradient stabil zu halten. Die maximale Korngröße liegt in Abhängigkeit der Fließeigenschaft der jeweils zu fördernden Flüssigkeit jeweils außerhalb einer die kapillare Förderung ausschließenden Größe. Anders ausgedrückt dürfen die Körner 24 nur so groß sein, dass sie als Dochtorgan 20 noch eine kapillare Wirkung erzeugen. In gleichem Maße darf der Korndurchmesser nicht kleiner als der Poren-/Kapillardurchmesser des Heizorgans 21 ausgebildet sein, um ein Verstopfen der Heizkapillaren und/oder Heraustreten der Körner 24 aus dem Heizorgan 21 zu verhindern.

Alle Körner 24 können aus demselben Material bestehen. Die Körner 24 können jedoch auch aus wenigstens zwei unterschiedlichen Materialien bestehen. Vorzugsweise bestehen die Körner 24 aus Sand (Quarz) und/oder Graphit. Als Materialien kommen aber auch diverse andere Materialien oder Materialmischungen in Frage. Bevorzugte Materialien für die Körner 24 sind z.B. PEEK-Granulat (Polyetheretherketon-Granulat), PEK-Granulat (Polyetherketon-Granulat), PA-Pulver, VM17-Granulat, Glas, Steatit, Siliziumdioxid, Lignin, Aerogel, Viton, Silikon, Asche, Charcoal, Betonit, Zeolith, Diatomit, Magnesiumsilikat, Korund, Kieselgur, gemahlener Porphyr sowie Mischungen daraus. Besonders bevorzugt bestehen die Körner 24 eines Dochtorgans 20 ausgehend vom Vorratstank 17 in Richtung des Strömungskanals 16 lokal aus unterschiedlichen Materialien. Als lokale Anordnung ist z.B. ein schichtweiser Aufbau von Körnern 24 aus jeweils gleichem Material zu verstehen.

Durch die Auswahl der Materialien der Körner 24 eines Dochtorgans 20 lassen sich verschiedene Eigenschaften des Dochtorgans 20 einstellen. Beispielsweise können Körner 24 mit unterschiedlichen Wärmeleitfähigkeiten eingesetzt werden. Besonders bevorzugt nimmt die Wärmeleitfähigkeit der Körner 24 ausgehend vom Vorratstank 17 in Richtung des Strömungskanals 16 kontinuierlich oder schritt- bzw. schichtweise zu. Eine besonders wärmeleitfähige Schicht kann z.B. im Grenzbereich zum Strömungskanal 16 gebildet sein, während in Richtung des Vorratstanks 17 eine Schicht mit nur geringer Wärmeleitfähigkeit gebildet ist. Die unterschiedliche Materialauswahl der Körner 24 führt auch dazu, dass die Körner 24 z.B. kompressibel ausgebildet sein können. In Abhängigkeit der Größe des Anpressdruckes, mit dem die Körner 24 z.B. in der Ausnehmung 31 des Trägerelemente 29 gehalten werden, kann durch elastische Verformung aktiv Einfluss auf die Größe der Poren einzelner Körner 24 oder benachbarter Körner 24 genommen werden. Der Verdampferkartusche 10 kann optional ein Stellorgan zugeordnet sein, mittels dem im Betriebszustand der Verdampferkartusche 10 der Anpressdruck auf das Dochtorgan 20 einstellbar ist. Das Stellorgan kann z.B. ein Hebelelement, ein Drehelement oder jedes andere Pressmittel sein.

Es lassen sich auch mehrschichtige Dochtorgane 20 bilden. In einer Ausführungsform kann eine erste Schicht mit Körnern 24 einer ersten Kornart gebildet sein. Eine zweite Schicht ist mit Körnern 24 einer zweiten Kornart gebildet. Eine dritte Schicht ist wieder mit der ersten Kornart gebildet. Die Körner 24 der zweiten Kornart in der mittleren Schicht weisen eine spezifische Eigenschaft auf, die z.B. mittels eines Mikrocontrollers der Steuereinheit 11 detektierbar ist. Während des Betriebs der Verdampferkartusche 10 führt z.B. eine Änderung der Benetzung der Körner 24 in der zweiten Schicht zu einer detektierbaren Änderung der spezifischen Eigenschaft der zweiten Kornart. Über den Mikrocontroller, der z.B. ein Sensor sein kann, wird diese Änderung detektiert. Mittels der Steuereinheit 11 kann dann in den Verdampfungsprozess regelnd eingegriffen werden, um z.B. einen so genannten Dry-Puff des Heizorganes 21 zu verhindern.

Die Körner 24 des Dochtorgans 20 können gleiche oder unterschiedliche geometrische Formen aufweisen. Die Körner 24 können beispielsweise nadelförmig, kugelförmig, in der Form eines Reiskorns oder auch dreieckig sein. Die Körner 24 können abgerundete Kanten aufweisen oder scharfkantig ausgebildet sein. Unter dem Begriff "Körner" werden ausdrücklich keine faserigen Elemente verstanden, also keine dünnen, feinen, fadenförmigen Gebilde. In Abhängigkeit der jeweiligen Form der Körner 24 und deren Korngröße können z.B. longitudinale und/ oder sphärische Poren gebildet sein. Die Poren können auch unregelmäßig ausgebildet sein. Die Körner 24 können auch mindestens teilweise magnetisch sein. Dadurch lassen sich die Körner 24 z.B. während des Befüllens/Schüttens in den Aufnahmeraum 25 bzw. die Ausnehmung 31 durch Anlegen eines externen Magnetfeldes in gewünschte Ausrichtungen ausrichten. Mit der Möglichkeit der Ausrichtung der Körner 24, z.B. können nadelförmige Körner 24 senkrecht zum Strömungskanal 16 ausgerichtet werden, lassen sich die Eigenschaften des Dochtorgans 20 individuell bestimmen, um das Dochtorgan 20 z.B. als Rückschlagventil oder als Steuerventil einsetzen zu können.

Erfindungsgemäß sind das Dochtorgan 20 und das Heizorgan 21 separate Einheiten, die in einem Kontaktbereich 35 aneinander liegen, wobei das Dochtorgan 20 dem Vorratstank 17 zugewandt ist und das Heizorgan 21 dem Strömungskanal 16 zugewandt ist. In dieser Variante weist das Heizorgan 21 elektrische Kontakte 22 zur elektrischen Kontaktierung mit der Energiequelle 12 auf. Am Beispiel der Ausführungsform gemäß Figur 6 ist die dann "zweiteilige", aus Dochtorgan 20 und Heizorgan 21 gebildete Verdampfereinheit 19 in der Ausnehmung 31 des Trägerelementes 29 angeordnet. Seitlich umlaufend ist die Verdampfereinheit 19 durch die Wandelemente 26 der Ausnehmung 31 eingefasst und gehalten. Zum Vorratstank 17 hin weist die Eintrittsseite E_{D} des Dochtorgans 20. Um zu verhindern, dass die losen oder miteinander verbundenen Körner 24 des Dochtorgans 20 ihre Position/Lage verlassen, ist das Dochtorgan 20 zum Vorratstank 17 hin gesichert. Die Sicherung kann auf unterschiedliche Weise erfolgen. Besonders einfach ist die mechanische Sicherung, beispielsweise durch das Deckelelement 27. Optional sind aber auch chemische, elektrostatische, pneumatische oder magnetische Sicherungen einsetzbar. Alle Sicherungen sind jedoch in Richtung des Vorratstanks 17 flüssigkeitspermeabel ausgebildet und gewährleisten die Flüssigkeitskopplung zwischen dem Vorratstank 17 und dem Heizorgan 21, das mit seiner Austrittsöffnung A_{H} hin zum Strömungskanal 16 weist. Das Dochtorgan 20 schirmt somit das Heizorgan 21 vom direkten Kontakt zum Vorratstank 17 ab.

Das Dochtorgan 20 weist Mikrokanäle 23 auf. Das Heizorgan 21 ist flüssigkeits- und dampfpermeabel ausgebildet. Das Dochtorgan 20 kann in einer der zuvor beschriebenen Ausführungsformen ausgebildet und eingerichtet sein. Mit seiner Austrittsseite A_{D} liegt das Dochtorgan 20 an der Eintrittsöffnung E_{H} des Heizorganes 21 an und bildet den Kontaktbereich 35. Das Heizorgan 21 selbst weist vorzugsweise lineare und/oder nicht lineare Durchgänge auf, die in den Strömungskanal 16 münden. Das Heizorgan 21 kann eine ebene oder gekrümmte oder anderweitig geformte Ausbildung aufweisen. Besonders bevorzugt ist das Heizorgan 21 ein im Wesentlichen aus Silizium bestehendes oder Silizium oder p- oder n-dotiertes Silizium aufweisendes MEMS-Bauteil (Micro-electro-mechanical-system), das ausgehend von einer dem Dochtorgan 20 zugewandten Oberseite bis zu einer dem Strömungskanal 16 zugewandten Unterseite flüssigkeits- und gas- bzw. dampfpermeable Durchgänge aufweist. Erfindungsgemäß ist die minimale Korngröße der Körner 24 des Dochtorgans 20 mindestens im Kontaktbereich 35 zum Heizorgan 21 größer als der mittlere Durchmesser der Durchgänge des Heizorganes 21.

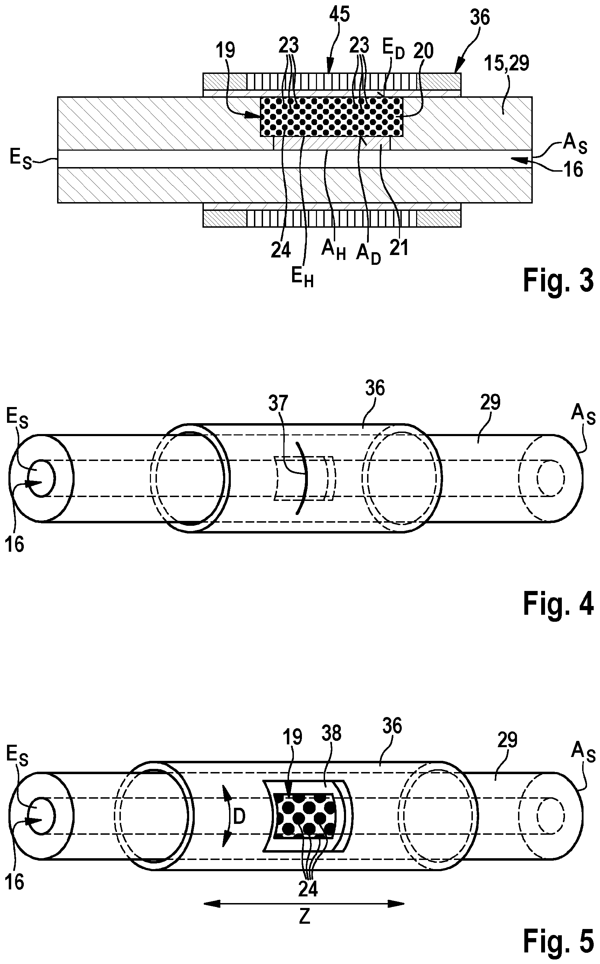

Wie zuvor beschrieben umfasst die Verdampferkartusche 10 ein Gehäuse 33, das den Hohlkörper 15 bzw. das Trägerelement 29 und die Verdampfereinheit 19 umgibt, wobei die Gehäusewand 34 den Vorratstank 17 zur Umgebung hin abgrenzt. Das Gehäuse 33 ist vorzugsweise zylindrisches oder stabförmig ausgebildet. Die Gehäusewand 34 kann beabstandet zum Dochtorgan 20 angeordnet sein. In anderen Ausführungsformen kann die Gehäusewand 34 das Dochtorgan 20 in seiner Position halten. Der Vorratstank 17 kann auch unabhängig und separat zum Gehäuse 33 ausgebildet sein. In den Figuren 3 bis 5 und 7 bis 12 werden weitere Ausführungsformen erfindungsrelevanter Merkmale beschrieben.

In der Figur 3 ist beispielsweise ein rohrförmiges Trägerelement 29 mit einer durchgängigen Durchgangsöffnung 30 dargestellt, das in der Ausnehmung 31 die zweiteilige Verdampfereinheit 19 trägt. Die Verdampfereinheit 19 ist durch eine elastische Hülse 36, die mindestens im Bereich des Dochtorgans 20 flüssigkeitspermeabel ausgebildet ist, hierzu kann die Hülse 36 z.B. eine Perforation 45 aufweisen, in der Ausnehmung 31 gehalten. Anstelle der elastischen Hülse 36 kann z.B. auch ein Wickel aus Vliesmaterial eingesetzt werden. In den Figuren 4 und 5 ist jeweils ein rohrförmiges Trägerelement 29 dargestellt, bei dem die Verdampfereinheit 19 ebenfalls durch eine elastische Hülse 36 in Position gehalten wird. In der Figur 4 deckt die Hülse 36 das Dochtorgan 20 ab. Die Hülse 36 weist jedoch einen Schlitz 37 auf, der sich durch Zug (siehe Pfeil Z) an der Hülse 36 zu einem Fenster 38 (siehe Figur 5) öffnet, um das Dochtorgan 20 freizugeben. Anstelle des Schlitzes 37 kann grundsätzlich das Fenster 38 vorgesehen sein, dass durch Drehen (siehe Pfeil D) der Hülse 36 aus einer Position, in der die Hülse 36 das Dochtorgan 20 abdeckt, in eine Position, in der das Fenster 38 über dem Dochtorgan 20 liegt, bewegbar ist.

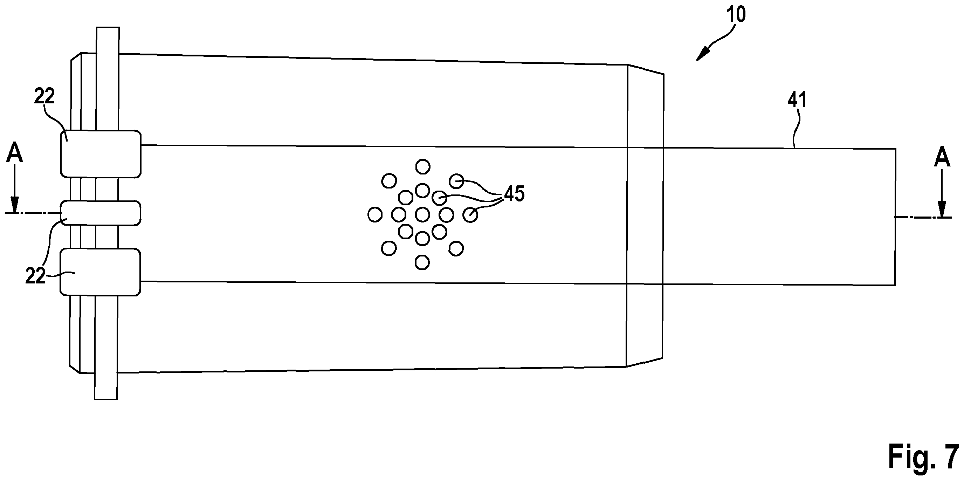

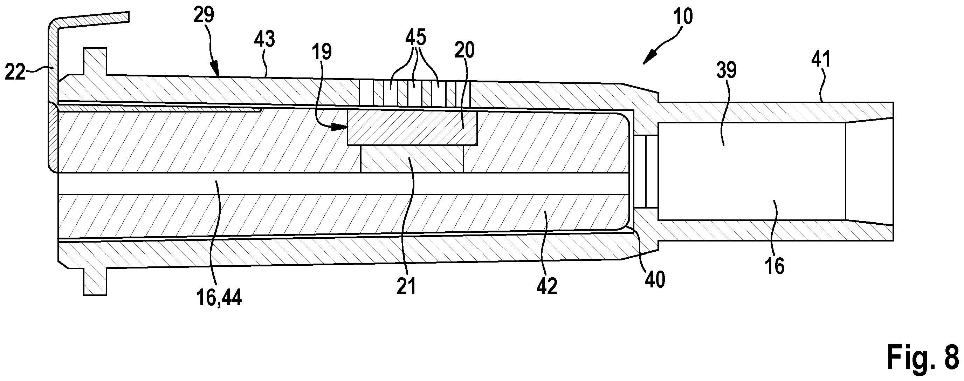

Die Figuren 7 und 8 zeigen eine Ausführungsform, bei der das Trägerelement 29 rohrförmig ausgebildet ist, wobei innerhalb der Durchgangsöffnung 30 zwei miteinander verbundene Kammern 39, 40 ausgebildet sind. Eine Kammer 39 dient als Mundstück 41. In der zweiten Kammer 40 ist ein Einsatz 42 angeordnet, der die Verdampfereinheit 19 mit dem Heizorgan 21 und dem Dochtorgan 20 trägt. Die Verdampfereinheit 19 ist zwischen dem Einsatz 42 und der Innenseite einer Wandung 43 des Trägerelementes 29 "eingeklemmt", also in Position gehalten. Der Einsatz 42 weist des Weiteren eine Durchgangsöffnung 44 auf, die mit der ersten Kammer 39 zur Bildung des Strömungskanals 16 in Wirkverbindung steht. Das Trägerelement 29 weist in der Wandung 43 in dem Bereich, an dem das Dochtorgan 20 an die Innenseite der Wandung 43 des rohrförmigen Trägerelementes 29 stößt, eine Perforation 45 auf, die eine Flüssigkeitskopplung zum Vorratstank 17 sicherstellt. Der Vorratstank 17 wird entsprechend zwischen der Gehäusewand 34 des Gehäuses 33 und der Wandung 43 des Trägerelementes 29 gebildet.

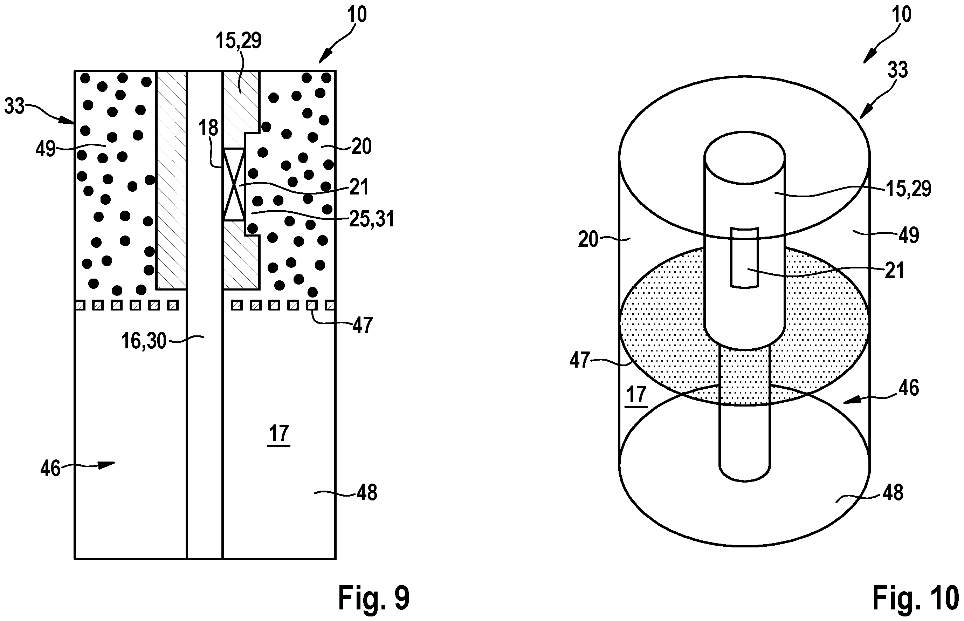

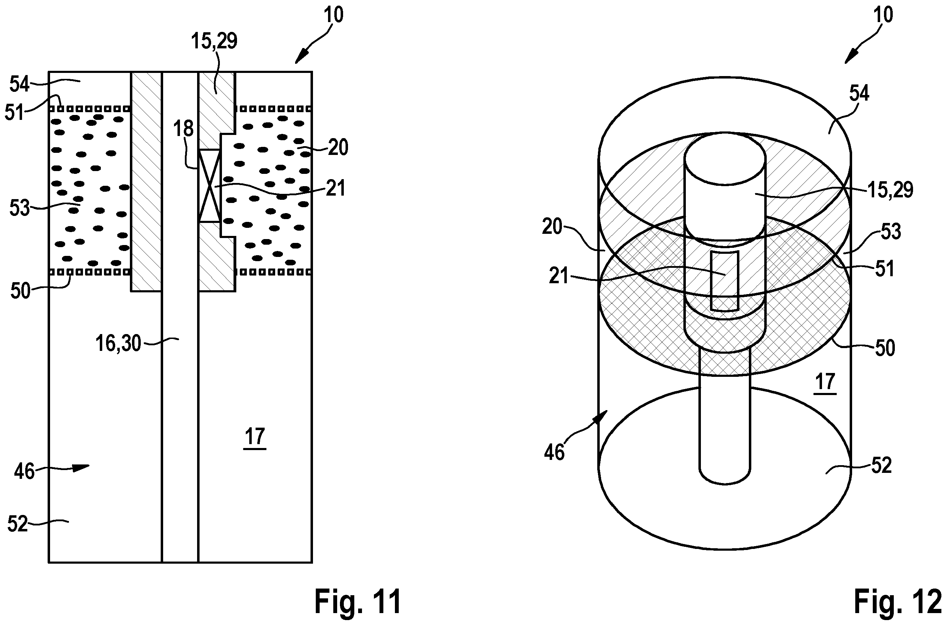

Die Figuren 9 bis 12 zeigen weitere Ausführungsformen möglicher Verdampferkartuschen 10. Bei diesen Ausführungsformen ist ein zylinderförmiges Gehäuse 33 zur Bildung eines Innenraums 46 vorgesehen, in dem als Strömungskanal 16 ein durchgängiger Schlot vorgesehen ist. Die Form des Gehäuses 33 kann jedoch beliebig sein. Das Trägerelement 29 erstreckt sich über einen Teil des Strömungskanals 16. In dem Trägerelement 29 ist die Ausnehmung 31 ausgebildet, in dem das Heizorgan 21 angeordnet ist. In der Variante der Figuren 9 und 10 teilt eine Gitterstruktur 47 den Innenraum 46 in zwei Bereiche 48, 49, wobei ein Bereich 48 als Vorratstank 17 für die Flüssigkeit und ein Bereich 49 zur Aufnahme des granularen Dochtorgans 20 dient. Die Gitterstruktur 47 ist unterhalb des Trägerelementes 29 angeordnet, derart, dass das Dochtorgan 20 das Trägerelement 29 vollständig umgibt und entsprechend auch das Heizorgan 21 abdeckt. In der Variante der Figuren 11 und 12 sind zwei Gitterstrukturen 50 und 51 vorgesehen, die den Innenraum in drei Bereiche 52, 53, 54 teilen. Die beiden Gitterstrukturen 50, 51 sind oberhalb und unterhalb der Ausnehmung 31 mit dem Heizorgan 21 angeordnet, derart, dass der mittlere Bereich 53, der zur Aufnahme des Dochtorgans 20 dient, das Trägerelement 29 mindestens im Bereich des Heizorganes 21 abdeckt. In den anderen Bereichen 52, 54 kann Flüssigkeit bevorratet werden.

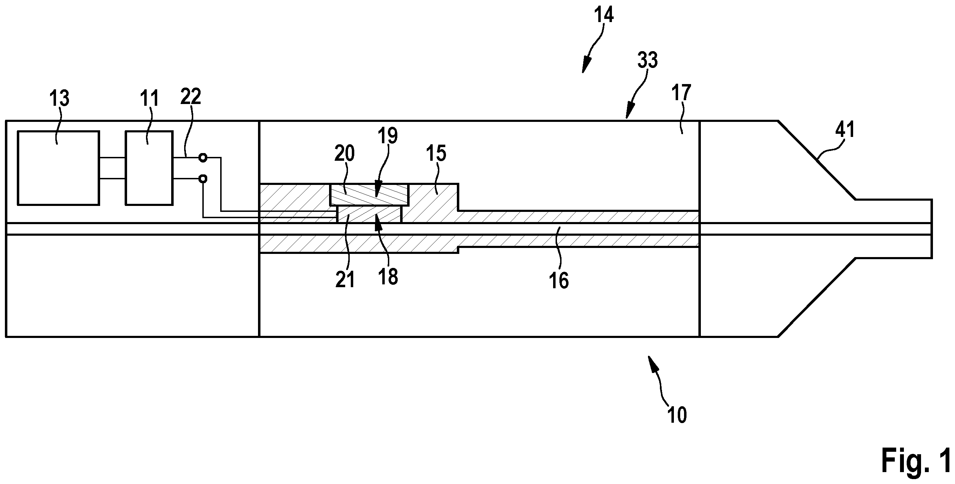

Das Funktionsprinzip des erfindungsgemäßen Inhalators 14, der eine Verdampferkartusche 10 gemäß der Erfindung umfasst, wird beispielhaft anhand einer E-Zigarette als Inhalator 14 insbesondere mit Bezug auf die Figur 1 beschrieben. Eine konsumierende Person saugt z.B. an einem Mundstück 41 des Inhalators 14, der aus dem Kartuschenträger 13 und der Verdampferkartusche 10 gebildet ist, wobei sich in dem Vorratstank 17 der Verdampferkartusche 10 eine Flüssigkeit befindet, die beispielsweise Glycerin, Propylenglycol und ggf. weitere Wirkstoffe und/oder Geschmacksstoffe enthält. Durch das Saugen wird in dem Strömungskanal 16 ein Unterdruck erzeugt, der seinerseits z.B. über einen nicht dargestellten Sensor die Steuereinheit 11 aktiviert. Die Steuereinheit 11 steuert das Heizorgan 21, das von der Energiequelle 12 mit Energie versorgt wird. Flüssigkeit aus dem Vorratstank 17 wird mittels des Dochtorgans 20 mindestens initial kapillar durch die Mikrokanäle 23 aus dem Vorratstank 17 in Richtung des Heizorganes 21 transportiert. Am bzw. im erwärmten Heizorgan 21 wird die Flüssigkeit zu Gas bzw. Dampf umgewandelt, wobei das Heizorgan 21 die Flüssigkeit bzw. das daraus gebildete Gas bzw. den daraus gebildeten Dampf aufgrund der flüssigkeits- und gas- bzw. dampfpermeablen Struktur in Richtung des Strömungskanals 16 transportiert und an diesen abgibt. Das aus dem Heizorgan 21 austretende Gas vermischt sich in dem Strömungskanal 16 mit dem Luftstrom, wobei es zu dem eigentlichen Rekondensations-/Dampfbildungsvorgang kommt, und wird von der konsumierenden Person angesaugt und inhaliert.

## Patentansprüche

1. Verdampferkartusche (10) als Bestandteil eines Inhalators, umfassend einen Hohlkörper (15) mit einem durchgängigen Strömungskanal (16) sowie einen Vorratstank (17) zum Bevorraten von Flüssigkeit, wobei der Vorratstank (17) mindestens eine Zugangsöffnung (18) zum Strömungskanal (16) aufweist und im Bereich jeder Zugangsöffnung (18) eine sich über die gesamte Zugangsöffnung (18) erstreckende Verdampfereinheit (19) angeordnet ist, die ein Dochtorgan (20) und ein Heizorgan (21) aufweist, wobei die Verdampfereinheit (19) flüssigkeitspermeabel ausgebildet ist, derart, dass Flüssigkeit mindestens initial kapillar aus dem Vorratstank (17) durch die Verdampfereinheit (19) in Richtung des Strömungskanals (16) förderbar ist, **dadurch gekennzeichnet, dass** das Dochtorgan (20) und das Heizorgan (21) separate Einheiten sind, die in einem Kontaktbereich (35) aneinanderliegen, wobei das Dochtorgan (20) dem Vorratstank (17) zugewandt ist und das Heizorgan (21) dem Strömungskanal (16) zugewandt ist, und das Heizorgan (21) elektrische Kontakte (22) aufweist, und wobei das Dochtorgan (20) Mikrokanäle (23) aufweist und das Heizorgan (21), das vom Dochtorgan (20) gegenüber dem Vorratstank (17) abschirmt ist, flüssigkeits- und dampfpermeabel ausgebildet ist, wobei das Dochtorgan (20) aus einer Vielzahl granulatartiger Körner (24) gebildet ist, die aufgrund ihrer Schüttung und/oder Ausbildung Mikrokanäle (23) bilden, wobei die minimale Korngröße der Körner (24) des Dochtorgans (20) mindestens im Kontaktbereich zum Heizorgan (21) größer ist als der mittlere Durchmesser der Durchgänge des Heizorgans (21).

2. Verdampferkartusche (10) nach Anspruch 1, **dadurch gekennzeichnet, dass** sich die Mikrokanäle (23) durchgängig von einer Eintrittsseite ED des Dochtorgans (20) zu einer Austrittsseite AD des Dochtorgans (20) erstrecken.

3. Verdampferkartusche (10) nach Anspruch 1 oder 2, **dadurch gekennzeichnet, dass** sie zum mechanischen und elektrischen Verbinden mit einem mindestens eine elektronische Steuereinheit (11) und eine Energiequelle (12) umfassenden Kartuschenträger (13) zur Bildung eines Inhalators (14) ausgebildet und eingerichtet ist, wobei die Verdampfereinheit (19) elektrische Kontakte (22) zur elektrischen Kontaktierung mit der Energiequelle (12) umfasst.

4. Verdampferkartusche (10) nach einem oder mehreren der Ansprüche 1 bis 3, **dadurch gekennzeichnet, dass** das das granulare Dochtorgan (20) bildende, körnige Material mindestens teilweise elektrisch leitfähig ist.

5. Verdampferkartusche (10) nach einem oder mehreren der Ansprüche 1 bis 4, **dadurch gekennzeichnet, dass** im Bereich der Zugangsöffnung (18) ein Aufnahmeraum (25) zum Aufnehmen der Verdampfereinheit (19) ausgebildet ist, wobei der Aufnahmeraum (25) zum Aufnehmen von Flüssigkeit aus dem Vorratstank (17) in die Verdampfereinheit (19) und zum Abgeben von gasförmigem Liquid/Dampf aus der Verdampfereinheit (19) in den Strömungskanal (16) mindestens teilweise durch eine flüssigkeits- und gas- bzw. dampfpermeable Struktur begrenzt ist.

6. Verdampferkartusche (10) nach einem oder mehreren der Ansprüche 1 bis 5, **dadurch gekennzeichnet, dass** die Verdampferkartusche (10) ein Trägerelement (29) umfasst, das den Hohlkörper (15) bildet und zum einen eine Durchgangsöffnung (30) zur Bildung des Strömungskanals (16) aufweist und zum anderen eine Ausnehmung (31) zur Aufnahme der Verdampfereinheit (19) aufweist.

7. Verdampferkartusche (10) nach einem oder mehreren der Ansprüche 4 bis 6, **dadurch gekennzeichnet, dass** ein aus dem granularen und elektrisch leitfähigen Material gebildetes Sediment fixiert ist, derart, dass das Sediment eine dreidimensionale Widerstandsheizmatrix mit parallelen und/oder in Reihe geschalteten Widerständen bildet.

8. Verdampferkartusche (10) nach einem oder mehreren der Ansprüche 1 bis 7, **dadurch gekennzeichnet, dass** die Körner (24) des Dochtorgans (20) hinsichtlich ihrer Materialauswahl und/oder ihrer Größe gleich und/oder ungleich ausgebildet sind.

9. Verdampferkartusche (10) nach einem oder mehreren der Ansprüche 1 bis 8, **dadurch gekennzeichnet, dass** die Körner (24) des Dochtorgans (20) ausgehend vom Vorratstank (17) in Richtung des Strömungskanals (16) lokal unterschiedliche Korngrößen aufweisen.

10. Verdampferkartusche (10) nach einem oder mehreren der Ansprüche 1 bis 9, **dadurch gekennzeichnet, dass** die Körner (24) des Dochtorgans (20) ausgehend vom Vorratstank (17) in Richtung des Strömungskanals (16) lokal aus unterschiedlichen Materialien bestehen.

11. Verdampferkartusche (10) nach einem oder mehreren der Ansprüche 5 bis 10, **dadurch gekennzeichnet, dass** die Körner (24) des Dochtorgans (20) als lose Schüttung innerhalb des Aufnahmeraums (25) liegen.

12. Verdampferkartusche (10) nach einem oder mehreren der Ansprüche 5 bis 10, **dadurch gekennzeichnet, dass** die Körner (24) des Dochtorgans (20) als miteinander verbundene Schüttung innerhalb des Aufnahmeraums (25) liegen.

13. Verdampferkartusche (10) nach einem oder mehreren der Ansprüche 1 bis 12, **dadurch gekennzeichnet, dass** die Korngröße zwischen 0,1 µm und 2mm, bevorzugt zwischen 3 und 300µm, beträgt.

14. Verdampferkartusche (10) nach einem oder mehreren der Ansprüche 1 bis 13, **dadurch gekennzeichnet, dass** die maximale Korngröße in Abhängigkeit der Fließeigenschaft der jeweils zu fördernden Flüssigkeit außerhalb einer die kapillare Förderung ausschließenden Größe liegt.

15. Verdampferkartusche (10) nach einem oder mehreren der Ansprüche 1 bis 14, **dadurch gekennzeichnet, dass** die Körner (24) des Dochtorgans (20) aus Sand und/oder Graphit bestehen.

16. Verdampferkartusche (10) nach einem oder mehreren der Ansprüche 1 bis 15, **dadurch gekennzeichnet, dass** die Körner (24) des Dochtorgans (20) mindestens teilweise magnetisch sind.

17. Verdampferkartusche (10) nach Anspruch 1, **dadurch gekennzeichnet, dass** das Heizorgan (21) ein im Wesentlichen aus Silizium bestehendes oder Silizium oder p- oder n-dotiertes Silizium aufweisendes MEMS-Bauteil (Micro-electro-mechanical-system) ist, das ausgehend von einer dem Dochtorgan (20) zugewandten Oberseite bis zu einer dem Strömungskanal (16) zugewandten Unterseite flüssigkeits- und gas- bzw. dampfpermeable Durchgänge aufweist.

18. Verdampferkartusche (10) nach einem oder mehreren der Ansprüche 1 bis 17, **dadurch gekennzeichnet, dass** sie ein Gehäuse (33) umfasst, das den Hohlkörper (15) und die Verdampfereinheit (19) umgibt, wobei eine Gehäusewand (34) des Gehäuses (33) den Vorratstank (17) zur Umgebung hin abgrenzt.

19. Verdampferkartusche (10) nach Anspruch 18, **dadurch gekennzeichnet, dass** die Gehäusewand (34) das Dochtorgan (20) in seiner Position hält.

20. Inhalator (14), ausgebildet und eingerichtet zum Inhalieren von mit Wirkstoffen angereichertem Dampf, umfassend einen mindestens eine elektronische Steuereinheit (11) und eine Energiequelle (12) umfassenden Kartuschenträger (13) sowie eine Verdampferkartusche (10), **dadurch gekennzeichnet, dass** die Verdampferkartusche (10) nach einem oder mehreren der Ansprüche 1 bis 19 ausgebildet und eingerichtet ist.

## Claims

1. Vaporizer cartridge (10) as a component of an inhaler, comprising a hollow body (15) with a continuous flow channel (16) as well as a storage tank (17) for storing liquid, wherein the storage tank (17) has at least one access opening (18) to the flow channel (16) and a vaporizer unit (19) extending over the entire access opening (18) is arranged in the region of each access opening (18) and has a wick member (20) and a heating member (21), wherein the vaporizer unit (19) is formed to be liquid-permeable such that liquid can be conveyed at least initially in a capillary manner out of the storage tank (17) through the vaporizer unit (19) in the direction of the flow channel (16), **characterized in that** the wick member (20) and the heating member (21) are separate units which lie against one another in a contact region (35), wherein the wick member (20) faces the storage tank (17) and the heating member (21) faces the flow channel (16), and the heating member (21) has electrical contacts (22), and wherein the wick member (20) has microchannels (23) and the heating member (21), which is shielded off from the storage tank (17) by the wick member (20), is formed to be liquid- and vapour-permeable, wherein the wick member (20) is formed from a plurality of granular grains (24) which form microchannels (23) as a result of their fill and/or formation, wherein the minimum grain size of the grains (24) of the wick member (20) at least in the contact region to the heating member (21) is larger than the average diameter of the passages of the heating member (21).

2. Vaporizer cartridge (10) according to Claim 1, **characterised in that** the microchannels (23) extend continuously from an entry side E_{D} of the wick member (20) to an exit side A_{D} of the wick member (20).

3. Vaporizer cartridge (10) according to Claim 1 or 2, **characterised in that** it is configured and adapted for mechanical and electrical connection to a cartridge carrier (13), for the formation of an inhaler (14), at least comprising an electronic control unit (11) and an energy source (12), wherein the vaporizer unit (19) comprises electrical contacts (22) for electrical contact with the energy source (12).

4. Vaporizer cartridge (10) according to one or more of Claims 1 to 3, **characterised in that** the granulous material which forms the granular wick member (20) is at least partially electrically conductive.

5. Vaporizer cartridge (10) according to one or more of Claims 1 to 4, **characterised in that** a receiving chamber (25) for receiving the vaporizer unit (19) is formed in the region of the access opening (18), wherein the receiving chamber (25) for receiving liquid from the storage tank (17) into the vaporizer unit (19) and to discharge gaseous liquid/vapour from the vaporizer unit (19) into the flow channel (16) is at least partially delimited by a liquid- and gas- or vapour-permeable structure.

6. Vaporizer cartridge (10) according to one or more of Claims 1 to 5, **characterised in that** the vaporizer cartridge (10) comprises a carrier element (29) which forms the hollow body (15) and on the one hand has a through-opening (30) for forming the flow channel (16) and on the other hand has a recess (31) for receiving the vaporizer unit (19).

7. Vaporizer cartridge (10) according to one or more of Claims 4 to 6, **characterised in that** a sediment formed from the granular and electrically conductive material is fixed such that the sediment forms a three-dimensional resistance heating matrix with parallel resistors and/or resistors connected in series.

8. Vaporizer cartridge (10) according to one or more of Claims 1 to 7, **characterised in that** the grains (24) of the wick member (20) are formed to be identical and/or non-identical in terms of their material selection and/or their size.

9. Vaporizer cartridge (10) according to one or more of Claims 1 to 8, **characterised in that** the grains (24) of the wick member (20), proceeding from the storage tank (17) in the direction of the flow channel (16), have locally different grain sizes.

10. Vaporizer cartridge (10) according to one or more of Claims 1 to 9, **characterised in that** the grains (24) of the wick member (20), proceeding from the storage tank (17) in the direction of the flow channel (16), are composed locally of different materials.

11. Vaporizer cartridge (10) according to one or more of Claims 5 to 10, **characterised in that** the grains (24) of the wick member (20) lie as loose fill within the receiving chamber (25).

12. Vaporizer cartridge (10) according to one or more of Claims 5 to 10, **characterised in that** the grains (24) of the wick member (20) lie as fill connected to one another within the receiving chamber (25).

13. Vaporizer cartridge (10) according to one or more of Claims 1 to 12, **characterised in that** the grain size is between 0.1µm and 2mm, preferably between 3 and 300µm.

14. Vaporizer cartridge (10) according to one or more of Claims 1 to 13, **characterised in that** the maximum grain size, depending on the flow property of the liquid to be conveyed in each case, lies outside a magnitude which rules out capillary conveyance.

15. Vaporizer cartridge (10) according to one or more of Claims 1 to 14, **characterised in that** the grains (24) of the wick member (20) are composed of sand and/or graphite.

16. Vaporizer cartridge (10) according to one or more of Claims 1 to 15, **characterised in that** the grains (24) of the wick member (20) are at least partially magnetic.

17. Vaporizer cartridge (10) according to Claim 1, **characterised in that** the heating member (21) is a MEMS component (Micro-Electro-Mechanical-System) which is composed substantially of silicon or has silicon or p- or n-doped silicon and, proceeding from an upper side facing the wick member (20) down to a lower side facing the flow channel (16), has liquid- and gas- or vapour-permeable passages.

18. Vaporizer cartridge (10) according to one or more of Claims 1 to 17, **characterised in that** it comprises a housing (33) which surrounds the hollow body (15) and the vaporizer unit (19), wherein a housing wall (34) of the housing (33) delimits the storage tank (17) towards the surroundings.

19. Vaporizer cartridge (10) according to Claim 18, **characterised in that** the housing wall (34) holds the wick member (20) in its position.

20. Inhaler (14), configured and adapted for the inhalation of vapour enriched with active ingredients, comprising a cartridge carrier (13) at least comprising an electronic control unit (11) and an energy source (12) as well as a vaporizer cartridge (10), **characterized in that** the vaporizer cartridge (10) is configured and adapted according to one or more of Claims 1 to 19.

## Revendications

1. Cartouche de vaporisation (10) en tant que constituant d'un inhalateur, comprenant un corps creux (15) avec un canal d'écoulement continu (16) ainsi qu'un réservoir de stockage (17) pour le stockage de liquide, le réservoir de stockage (17) présentant au moins une ouverture d'accès (18) au canal d'écoulement (16) et une unité de vaporisation (19) s'étendant sur toute l'ouverture d'accès (18) étant agencée dans la zone de chaque ouverture d'accès (18), qui présente un organe formant mèche (20) et un organe de chauffage (21), l'unité de vaporisation (19) étant configurée de manière à être perméable aux liquides, de telle sorte que du liquide peut être transporté au moins initialement par capillarité depuis le réservoir de stockage (17) à travers l'unité de vaporisation (19) en direction du canal d'écoulement (16), **caractérisée en ce que** l'organe formant mèche (20) et l'organe de chauffage (21) sont des unités séparées, qui s'appliquent l'une contre l'autre dans une zone de contact (35), l'organe formant mèche (20) étant tourné vers le réservoir de stockage (17) et l'organe de chauffage (21) étant tourné vers le canal d'écoulement (16), et l'organe de chauffage (21) présentant des contacts électriques (22), et l'organe formant mèche (20) présentant des microcanaux (23) et l'organe de chauffage (21), qui est masqué par l'organe formant mèche (20) par rapport au réservoir de stockage (17), étant configuré pour être perméable aux liquides et à la vapeur, l'organe formant mèche (20) étant formé d'une pluralité de grains (24) de type granulat qui forment des microcanaux (23) en raison de leur remplissage et/ou de leur configuration, la taille de grain minimale des grains (24) de l'organe formant mèche (20) étant supérieure au diamètre moyen des passages de l'organe de chauffage (21) au moins dans la zone de contact avec l'organe de chauffage (21).

2. Cartouche de vaporisation (10) selon la revendication 1, **caractérisée en ce que** les microcanaux (23) s'étendent de manière continue d'un côté entrée ED de l'organe formant mèche (20) à un côté sortie AD de l'organe formant mèche (20).

3. Cartouche de vaporisation (10) selon la revendication 1 ou 2, **caractérisée en ce qu'**elle est configurée et adaptée pour être reliée mécaniquement et électriquement à un support de cartouche (13) comprenant au moins une unité de commande électronique (11) et une source d'énergie (12) pour former un inhalateur (14), l'unité de vaporisation (19) comprenant des contacts électriques (22) pour la mise en contact électrique avec la source d'énergie (12).

4. Cartouche de vaporisation (10) selon une ou plusieurs des revendications 1 à 3, **caractérisée en ce que** le matériau granuleux qui forme l'organe formant mèche granulaire (20) est au moins partiellement conducteur électrique.

5. Cartouche de vaporisation (10) selon une ou plusieurs des revendications 1 à 4, **caractérisée en ce qu'**un espace de réception (25) destiné à recevoir l'unité de vaporisation (19) est configuré dans la zone de l'ouverture d'accès (18), l'espace de réception (25) destiné à recevoir du liquide du réservoir de stockage (17) dans l'unité de vaporisation (19) et à évacuer du liquide gazeux/de la vapeur de l'unité de vaporisation (19) dans le canal d'écoulement (16) étant au moins partiellement délimité par une structure perméable aux liquides et aux gaz ou à la vapeur.

6. Cartouche de vaporisation (10) selon une ou plusieurs des revendications 1 à 5, **caractérisée en ce que** la cartouche de vaporisation (10) comprend un élément de support (29) qui forme le corps creux (15) et qui présente d'une part une ouverture de passage (30) pour former le canal d'écoulement (16) et d'autre part un évidement (31) pour recevoir l'unité de vaporisation (19).

7. Cartouche de vaporisation (10) selon une ou plusieurs des revendications 4 à 6, **caractérisée en ce qu'**un sédiment formé par le matériau granulaire et conducteur électrique est fixé, de telle sorte que le sédiment forme une matrice de chauffage résistive tridimensionnelle avec des résistances en parallèle et/ou en série.

8. Cartouche de vaporisation (10) selon une ou plusieurs des revendications 1 à 7, **caractérisée en ce que** les grains (24) de l'organe formant mèche (20) sont configurés identiques et/ou non identiques en ce qui concerne leur choix de matériau et/ou leur taille.

9. Cartouche de vaporisation (10) selon une ou plusieurs des revendications 1 à 8, **caractérisée en ce que** les grains (24) de l'organe formant mèche (20) présentent localement des tailles de grains différentes en partant du réservoir de stockage (17) en direction du canal d'écoulement (16).

10. Cartouche de vaporisation (10) selon une ou plusieurs des revendications 1 à 9, **caractérisée en ce que** les grains (24) de l'organe formant mèche (20) sont localement constitués de matériaux différents en partant du réservoir de stockage (17) en direction du canal d'écoulement (16).

11. Cartouche de vaporisation (10) selon une ou plusieurs des revendications 5 à 10, **caractérisée en ce que** les grains (24) de l'organe formant mèche (20) se présentent sous forme de remplissage lâche à l'intérieur de l'espace de réception (25).

12. Cartouche de vaporisation (10) selon une ou plusieurs des revendications 5 à 10, **caractérisée en ce que** les grains (24) de l'organe formant mèche (20) se présentent sous forme de remplissage interconnecté à l'intérieur de l'espace de réception (25).

13. Cartouche de vaporisation (10) selon une ou plusieurs des revendications 1 à 12, **caractérisée en ce que** la taille de grains est comprise entre 0,1 µm et 2 mm, de préférence entre 3 et 300 µm.

14. Cartouche de vaporisation (10) selon une ou plusieurs des revendications 1 à 13, **caractérisée en ce que** la taille de grains maximale se situe, en fonction des propriétés d'écoulement du liquide respectif à transporter, en dehors d'une taille excluant le transport par capillarité.

15. Cartouche de vaporisation (10) selon une ou plusieurs des revendications 1 à 14, **caractérisée en ce que** les grains (24) de l'organe formant mèche (20) sont constitués de sable et/ou de graphite.

16. Cartouche de vaporisation (10) selon une ou plusieurs des revendications 1 à 15, **caractérisée en ce que** les grains (24) de l'organe formant mèche (20) sont au moins partiellement magnétiques.

17. Cartouche de vaporisation (10) selon la revendication 1, **caractérisée en ce que** l'organe de chauffage (21) est un composant MEMS (système micro-électro-mécanique) constitué essentiellement de silicium ou présentant du silicium ou du silicium dopé p ou n, qui présente des passages perméables aux liquides et aux gaz ou à la vapeur en partant d'un côté supérieur tourné vers l'organe formant mèche (20) jusqu'à un côté inférieur tourné vers le canal d'écoulement (16).

18. Cartouche de vaporisation (10) selon une ou plusieurs des revendications 1 à 17, **caractérisée en ce qu'**elle comprend un boîtier (33) entourant le corps creux (15) et l'unité de vaporisation (19), une paroi de boîtier (34) du boîtier (33) délimitant le réservoir de stockage (17) par rapport à l'environnement.

19. Cartouche de vaporisation (10) selon la revendication 18, **caractérisée en ce que** la paroi de boîtier (34) maintient l'organe formant mèche (20) dans sa position.

20. Inhalateur (14), configuré et adapté pour inhaler de la vapeur enrichie en principes actifs, comprenant un support de cartouche (13) qui comprend au moins une unité de commande électronique (11) et une source d'énergie (12), ainsi qu'une cartouche de vaporisation (10), **caractérisé en ce que** la cartouche de vaporisation (10) est configurée et adaptée selon une ou plusieurs des revendications 1 à 19.
